# EUROPEAN PATENT SPECIFICATION

(11) **EP 0 463 126 B1**
(45) Date of publication and mention of the grant of the patent: **16.08.2000**
(21) Application number: 91900101.6
(22) Date of filing: 13.11.1990
(51) Int. Cl.: A61K 33/24

(54) **METHODS OF ENHANCING WOUND HEALING AND TISSUE REPAIR**
VERFAHREN ZUR VERBESSERUNG DER WUNDHEILUNG UND GEWEBEERNEUERUNG
PROCEDES POUR ACTIVER LA CICATRISATION DES PLAIES ET LA REPARATION DES TISSUS

(30) Priority: 12.01.1990 US 464361
(43) Date of publication of application: 02.01.1992
(60) Divisional application: 99118097.7
(73) Proprietor: NEW YORK SOCIETY FOR THE RELIEF OF THE RUPTURED AND CRIPPLED MAINTAINING THE HOSPITAL FOR SPECIAL SURGERY, New York, N.Y. 10021 (US)
(72) Inventor: BOCKMAN, Richard, New York, NY 10021 (US); GUIDON, Peter, Union, New Jersey 07083 (US)
(74) Representative: Perry, Robert Edward
(86) International application number: US9006606
(87) International publication number: WO9110437

(56) References cited:
- US-A- 4 529 593
- US-A- 4 704 277
- DATABASE WPIL Section Ch, Week 8746, Derwent Publications Ltd., London, GB; Class D22, AN 87-32445 & JP-A-62 230648 (ASAHI GLASS KK) 9 October 1987

## Description

### Background of the Invention

Recently, naturally produced substances have been discovered which promote growth and healing of connective and support tissues. Such substances have been termed growth factors. Growth factors, usually proteins, initiate programs of differentiation and/or development within an organism.

Although growth factors would appear to be ideal for inducing support and connective tissue repair, such factors are not practically useful as pharmaceutical agents. Growth factors are not stable and tend to break down upon storage. As proteins, growth factors are not suitable for oral administration since they are digested and destroyed before entering the blood stream. Since they are only slowly absorbed by the body and rapidly broken down, growth factors cannot be satisfactorily administered as topical ointments. As a result of the lability of growth factors the preferred route of administration is parenteral thus requiring medically supervised administration. Unfortunately, many of the growth factors are species-specific and are recognized as foreign by other species. Thus, there is the constant danger of eliciting an immune response. Lastly, there is no evidence that growth factors, when administered parenterally, target to skin, connective and support tissues.

With the exception of certain recently described naturally occurring factors, there is no disclosure or suggestion in the prior art of a pharmaceutically acceptable compound that can induce skin, connective and support tissues to synthesize new matrix components in a manner that simulates natural conditions of growth, healing and repair. Additionally, there are no reports of pharmaceutically acceptable compounds that normalise the function of matrix-forming cells and result in the production of new, normal skin, connective and support tissue components that enhance tissue repair and augmentation.

US-A-4529593, US-A-4686104 and US-A-4704277 describe methods of preventing excessive loss of calcium from human bone by the administration of pharmaceutically acceptable gallium-containing compounds. The ability of gallium-containing compounds to inhibit bone resorption (breakdown) prevents disordered calcium homeostasis.

### Summary of the Invention

According to the present invention, a gallium compound is used for the manufacture of a medicament capable of providing gallium in soluble form, for use in enhancing connective and support tissue repair and augmentation, provided that the tissue is not bone.

### Description of the Invention

It has now been found that pharmaceutically acceptable gallium-containing compounds such as gallium nitrate mimic the molecular action of transforming growth factor-β (TGF-β) with respect to inducing the synthesis of both mRNA and proteins involved in wound healing. In particular, results have been obtained, using gallium nitrate-treated skin cells, which indicate that the mechanism of action of gallium is similar to that of TGF-β in wound healing.

Mechanisms of wound healing are similar for skin, connective and support tissues. The tissues being discussed include not only skin, cartilage and tendon, but also those tissues containing a supporting fascia made up of collagen matrix that encapsulate muscles and organs. Although wound-healing requires a complex series of events that are not well defined, it is known that growth factors are required. Such factors are released into the wounded area, and migrate into the wound; there the factors stimulate growth of keratinocytes and fibroblasts, initiate angiogenesis and stimulate matrix formation and remodeling of the wounded area. ten Dijke et al., "Growth Factors for Wound Healing", Bio/Technology, 7:793-798 (1989).

From the data presented below it is evident that gallium-containing compounds mimic the action of TGF-β by directly stimulating the activity of matrix-forming cells and consequently enhancing the formation of matrix components. Matrix-forming cells synthesizing the structural elements of connective and support tissues respond to gallium-containing compounds in a manner similar to that of TGF-β, the growth factor-like effects of these compounds enhance the repair or augment the strength of skin, connective and support tissues. The mechanisms of wound healing are similar in many respects for man and animals; hence veterinary applications of this invention are also apparent.

Wound healing involving skin is an example of tissue repair. One aspect of wound healing in skin involves the production of critical matrix components that form the architectural and structural lattice of skin. The major matrix component is type I collagen, similar to the case in bone. In the skin, it is the fibroblast that synthesizes and releases new collagen into the wound site. Unlike bone, this matrix is not mineralized. Other proteins produced by fibroblasts contribute to the matrix. One such protein, fibronectin, is thought to function as an important anchoring protein, helping to bind key cells to the underlying matrix. Fibronectin, the synthesis of which is enhanced during wound repair, is also produced by fibroblasts.

In skins, the effects of gallium-containing compounds mimic the effects of TGF-β, a factor known to be involved in wound healing of skin, support and connective tissues throughout the body. Gallium-containing compounds are thus useful in enhancing skin healing and the healing of other connective and support tissues as well as augmenting their mass by the synthesis of proteins associated with wound healing even in the absence of a specific wound. Gallium-containing compounds, by virtue of their ability to mimic natural growth factors, are suitable for use in inducing the formation of skin, connective tissue matrix elements and thus to augment the strength of all connective and support tissues as well as to repair specific injuries (wounds) to such tissues.

A further advantage of the present invention is that gallium-containing compounds can be applied to the site of injury. In some cases, gallium shows a preference to accumulate in specific connective tissue, such as skin, offering the advantage that the agent can target to tissues in which it has beneficial effects. Previous teachings suggested that gallium-containing compounds had to be administered parenterally, thus necessitating hospitalization and the administration of relatively high concentrations of gallium. According to the present invention, gallium-containing compounds can now be applied directly to the site of injury or to the region requiring augmentation, resulting in lower serum, organ and non-involved tissue levels of gallium with increased effectiveness at the site of injury, and a concomitant decrease in the risk of side effects. Thus the preferred method of application is topical.

The effective amount and method of administration of the particular gallium formulation may vary based on the nature of the condition and disorder being treated, its severity, the age of the patient and other factors evident to one skilled in the art. In general, gallium-containing compounds are pharmaceutically acceptable due to their low toxicity in the therapeutic dosage range, stability and ability to be incorporated into a wide variety of vehicles for numerous routes of administration.

Gallium-containing compounds are useful in the art of tissue implants. Tissue implants include but are not limited to cartilage grafts, tendon grafts and skin grafts. The grafts can be selectively coated with topical applications containing gallium-containing compounds or impregnated by soaking or immersion in solutions of gallium-containing compounds prior to their implantation. In connective and support tissues, other than bone, gallium-containing compounds enhance tissue formation so as to promote wound healing and facilitate incorporation of grafts such as tendon, cartilage and skin.

In skin, topical or subcutaneous administration of gallium-containing compounds enhances new skin matrix synthesis. Increased production of the connective matrix components would facilitate healing of tears, breaks or defects in skin. Increased matrix synthesis would increase skin thickness, thereby removing wrinkles due to aging. Gallium-induced matrix synthesis could be used to selectively fill skin defects due to prior injury as from acne or previous trauma. Thus in addition to the uses in wound repair, gallium compounds could have many dermatologic and cosmetic uses for the treatment of skin disorders.

Gallium-containing compounds, especially gallium nitrate, in a pharmaceutically acceptable form and at doses far below those known to be cytotoxic have now been found to exert beneficial effects on osteoblasts, fibroblasts and keratinocytes. In explanted tissues, treatment with gallium nitrate has been found to enhance the synthesis of new connective and support tissue matrix elements.

According to the present invention, in order to obtain the beneficial effects of gallium, pharmaceutically acceptable gallium-containing compounds are administered to the patient in an amount sufficient to provide therapeutic levels of elemental gallium. Typically in the case of wound healing, therapeutic levels are attained when elemental gallium is present in a steady state concentration in blood of about 1-150 µM with a preferred range of about 1-50 µM or when elemental gallium is present in tissues at a steady state concentration of about 1-1000 ng/mg dry weight of tissue with a preferred range of about 1-500 ng/mg dry weight of tissue. However, if gallium-containing compounds are applied proximate to the site of injury, lower levels of gallium may be maintained within the body. For instance the effective level of gallium needed at the site of injury would be about 0.25-100 µM thereby resulting in limited levels of gallium in the blood and viscera since absorption from the site would be relatively insignificant. Administration of gallium-containing compounds would cease once wound healing has occurred. In the case of skin augmentation, lower concentrations of gallium-containing compounds are necessary and administration can be continuous for instance as a face cream applied daily.

Gallium-containing compounds are useful in formulating a variety of routes of administration. The route(s) of administration useful in a particular application are apparent to one of skill in the art. Routes of administration include but are not limited to topical, transdermal, parenteral, gastrointestinal, transbronchial and transalveolar.

Formulations of gallium-containing compounds suitable to be applied topically include but are not limited to implants, ointments, creams, rinses and gels. Formulations suitable for transdermal administration include but are not limited to suspensions, oils, creams and ointments applied directly or attached to a protective carrier such as a patch. Formulations suitable for parenteral administration include but are not limited to sterile solutions for intravenous, intramuscular, intraarticular or subcutaneous injection or infusion. Formulations suitable for gastrointestinal administration include, but are not limited to, pills or liquids for ingesting and suppositories for rectal administration. Formulations suitable for transbronchial and transalveolar administration include, but are not limited to various types of aerosols for inhalation. The above-mentioned formulations are meant to describe but not limit the methods of administering gallium-containing compounds. The methods of making the various formulations are within the ability of one skilled in the art and will not be described in detail here.

In skin, gallium-containing compounds are indicated for use in treatment of tears, breaks, fractures, implants and inadequate connective tissue mass.

Gallium-containing compounds are suitable for the treatment of wound healing involving the skin. Gallium compounds are useful in dermatologic conditions that involve the skin. In either circumstance, gallium-containing compounds are capable of inducing new skin matrix synthesis thereby accelerating healing. In addition to the benefits during wound repair, gallium-induced matrix augmentation in the skin can have beneficial cosmetic effects as in the repair of skin defects due to the loss of dermal elements necessary for maintaining tissue fullness and smoothness.

Gallium-containing compounds are useful in the treatment of wounds in connective and support tissue other than bone. Such tissue includes cartilage, tendon, collagen containing tissues that encapsulate organs and fascia. Collagen-containing tissues that encapsulate organs include but are not limited to the renal and liver capsules, the meninges, the pericardium and the pleura. The so-called soft tissues of skin, muscle and organs are supported by a collagen containing connective tissue known as fascia, and it is the fascia that is essential for the structural integrity and wound healing of these tissues.

In treatment of injury to connective and support tissue, administration of gallium-containing compounds proximate to the wound is preferred. For instance, cuts, abrasions and burns can be topically coated with ointments, creams, rinses or gels, or by the use of transdermal patches or by subcutaneous administration. In treatment of injuries to skin, tendon, cartilage and collagen containing tissues that encapsulate organs, topical application of gallium-containing compounds is preferred, and the preferred routes of administration are topical and transdermal. Adequate levels of gallium can also be obtained by systemic administration such as parenteral, gastrointestinal, transbronchial or transalveolar.

Additionally, tendon, cartilage, fascia and skin grafts are commonly used to treat torn or damaged tissue. These grafts can be coated with, soaked or immersed, in rinses or gels containing gallium-containing compounds prior to implantation. Topical administration of gallium-containing compounds is also useful to promote healing of tendon, cartilage and skin grafts. The gallium-containing compounds induce matrix component synthesis to enhance attachment, fixation and stabilization of implants by promoting new tissue growth onto the implant.

It has previously been shown that the active ingredient in gallium-containing compounds is the elemental gallium itself and not any accompanying salt. Therefore any compound which provides adequate blood and tissue levels or tissue levels proximate to the site of injury of elemental gallium can be used according to the present invention. Gallium nitrate has been used in the following examples and is representative of all pharmaceutically acceptable gallium-containing compounds capable of providing therapeutically effective levels of elemental gallium for uptake by the patient. Use of the term "gallium-containing compound" denotes one or more pharmaceutically acceptable compounds capable of supplying therapeutic levels of elemental gallium. Such compounds include but are not limited to gallium nitrate, gallium phosphate, gallium citrate, gallium chloride, gallium fluoride, gallium carbonate, gallium formate, gallium acetate, gallium tartrate, gallium maltol, gallium oxalate, gallium oxide, and hydrated gallium oxide. Coordination complexes of gallium including but not limited to gallium pyrones (such as those derived from kojic acid); gallium pyridones (such as desferal); gallium hydroxymates; gallium aminocarboxylates or gallium oximes (such as 8-hydroxy quinolone) are also included in the present invention. Gallium bound to proteins such as but not limited to transferrin or lactoferrin illustrates another class of gallium-containing compounds that are included in the present invention. Gallium is bound to proteins by means known in the art.

The present invention is further illustrated by the following specific examples, which are not intended in any way to limit the scope of the invention.

### Example 1

### The Effects of Gallium-Containing Compounds on Messenger RNA Levels in Human Fibroblast and Rat Osteoblast Cell Lines

The cell lines used in this study include human skin fibroblasts and a rat osteogenic sarcoma tumor cell line (ROS 17/2.8). The fibroblasts are primary cultures derived from human skin. The rat osteosarcoma cell line is a permanent cell line that maintains many of the characteristics of osteoblasts. Majeska et al., "Parathyroid Hormone: Responsive Clonal Cell Lines from Rat Osteosarcoma", Endocrinol., 107:1494-1503 (1980). To ascertain the specificity of gallium-containing compounds, and compare the compounds to TGF-β, the cells were administered several metal and near metal containing compounds, TGF-β or selected bisphosphonate compounds. The metal and near metal compounds included ferric nitrate, ferric chloride, ferrous chloride, aluminum chloride, zinc chloride, gallium nitrate, cisplatin and spirogermanium. The metal and near metal compounds were tested at 50 µM. The selected bisphosphonates included EHDP and clodronate (Cl₂MDP) were administered at concentrations known to have antiresorptive activity. TGF-β was tested at 5.0 ng/ml. The fibroblasts and osteoblasts were exposed to the compounds for 24-48 hrs., after which total cellular RNA was isolated by guanidinium isothiocyanate extraction and ultracentrifugation through cesium chloride. Chirgwin et al., "Isolation of Biologically Active Ribonucleic Acid from Sources Enriched in Ribonuclease", Biochem., 18:5294-5299 (1979). The RNA was separated by electrophoresis on a denaturing MOPS-formaldehyde agarose gel, (Biorad Products, Rockville Center, NY) transferred to Nytran filters (Schleicher and Schuell, Keane, NH) and hybridized with [³²P]dCTP (New England Nuclear) labeled cDNA probes specific for the matrix proteins. The probes included: α₁(I) procollagen; osteocalcin; osteonectin; osteopontin; fibronectin; and constitutively synthesized proteins such as tubulin and α-actin as controls. Radiolabeling of the probes was done according to the random priming method of Feinberg et al., "A Technique for Radiolabeling DNA Restriction Endonuclease Fragments to High Specific Activity", Anal. Biochem., 132:6-13 (1983). These probes were obtained from Drs.: David Rowe (α₁(I) procollagen, University of Connecticut, Storrs, CT); P. Robey (osteopontin, osteonectin, National Institutes of Health, Bethesda, MD); John Wozney (osteocalcin, Genetics Institute, Cambridge Park, MA); R. Hynes (fibronectin, Rockefeller University, New York, N.Y.); and Lydia Pan (α-actin, Stanford, Palo Alto, CA). Hybridization of the probes to the filters was performed according to the manufacturer's instructions. The hybridized filters (Northern blots) were washed under stringent conditions and exposed to X-ray film.

After developing the X-ray film, the bands corresponding to procollagen, fibronectin, osteopontin, osteocalcin and α-actin mRNA's were scanned to provide an estimate of the percent change in mRNA levels after gallium nitrate treatment compared to control cells not treated with gallium nitrate. The X-ray films were scanned using an LKB Model 2202 Ultroscan Laser densitometer. The numbers shown in Table 1 represent the percent change compared to untreated cells.

The results, listed in Table 1, showed that only the gallium nitrate and TGF-β caused an increase in α₁(I) procollagen, osteonectin and fibronectin mRNA with a concomitant decrease in osteocalcin. This result shows that only gallium nitrate mimics the changes seen when these same cells are exposed to TGF-β. Surprisingly, both ferric chloride and zinc chloride caused approximately two- to three-fold elevated levels of osteocalcin mRNA, an effect opposite to that of gallium or TGF-β.

**TABLE 1**

| PERCENT CHANGE IN MATRIX PROTEIN mRNA LEVELS AFTER GALLIUM TREATMENT | | | |
|---|---|---|---|
| mRNA | Gallium Nitrate | TGF-β | Zinc Chloride |
| | | | |

| OSTEOBLASTS | | | |
|---|---|---|---|
| α₁(I) Procollagen | +400 | +160 | - |
| Osteopontin | 0 | + 70 | - |
| Osteocalcin | - 40 | - 40 | +270 |
| α-Actin | + 10 | - | - |

| FIBROBLASTS | | | |
|---|---|---|---|
| α₁(I) Procollagen | +400 | | |
| Fibronectin | +350 | | |

| mRNA | Ferric Chloride | Aluminum Chloride | EHDP or Cl₂MDP |
|---|---|---|---|
| | | | |

| OSTEOBLASTS | | | |
|---|---|---|---|
| α₁(I) Procollagen | - | - | 0 |
| Osteopontin | - | - | - |
| Osteocalcin | +180 | 0 | 0 |
| α-Actin | - | - | - |

| FIBROBLASTS | | | |
|---|---|---|---|
| α₁(I) Procollagen | | | |
| Fibronectin | | | |

Thus gallium nitrate, but not other metal- or near metal-containing compounds or bisphosphonates, increases the synthesis of mRNA encoding key stromal matrix components. There was no evidence for an increase in TGF-β activity in the culture media of cells exposed to gallium nitrate, suggesting that the matrix enhancing effect was directly mediated by gallium nitrate, not indirectly through induction of TGF-β synthesis. Additionally, several other metals appear to induce production of osteocalcin which would result in decreased matrix formation.

### Example 2

### Gallium Effect on Human Keratinocyte Proliferation

A critical step in wound healing of skin tears and breaks is the re-epithelization of the wound. An initial step in wound healing is the proliferation of specific skin cells known as keratinocytes. These cells grow into a wound from the edges to provide a protective cellular barrier over the wound. At the present time, the endogenous natural growth factors in skin that can initiate this critical proliferation have not been fully identified. When freshly isolated human keratinocytes were treated with gallium nitrate, low concentrations of gallium induced a proliferative response.

Human keratinocytes were prepared from split thickness skin specimens removed from cadavers, as previously described, Staino-Coico et al., "Human Keratinocyte Culture", J. Clin. Invest., 77:396-404 (1986). Briefly, the tissue specimens were suspended in sterile Eagle's minimum essential medium containing antibiotics to prevent bacterial and fungal growth. The tissue was placed in phosphate-buffered saline that contained 0.5% trypsin but was calcium and magnesium free, for 90 minutes at 37°C. Single cell suspensions were prepared by the addition of 0.25% DNase I, then fetal calf serum followed by filtration through sterile gauze. The isolated cells were harvested by centrifugation then resuspended in media containing 20% fetal bovine serum supplemented with amino acids, hormones and antibiotics. All reagents and media were obtained from Sigma Chemical Company, St. Louis, MO.

By measuring keratinocyte cell numbers, it was shown that a 10µM concentration was sufficient to produce a doubling in keratinocyte cell number after seven days. The results are shown in Table 2 where the cell count is in the millions and the control is untreated cells.

**TABLE 2**

| Effect of Gallium on Human Keratinocytes | | |
|---|---|---|
| Day of Study | Control | Gallium |
| 3 | 3.12 ± 0.58 | 3.45 ± 0.40 |
| 7 | 2.80 ± 0.81 | 5.33 ± 0.15 |

Increasing keratinocyte proliferation means that wound healing is accelerated. The protective barrier over the skin wound is thus established earlier allowing subsequent healing to proceed in a protected and sterile environment. Wound infection due to inadequate sealing of the wound is probably one of the major impediments to wound healing. Coupled with known enhancement of fibroblast production of key matrix components, gallium-containing compound treatment of skin wounds greatly facilitates and accelerates wound healing.

## Claims

1. Use of a gallium compound for the manufacture of a medicament capable of providing gallium in soluble form, for enhancing connective and support tissue repair and augmentation, provided that the tissue is not bone.

2. Use according to claim 1, wherein the gallium compound is gallium nitrate, gallium citrate, gallium phosphate, gallium chloride, gallium fluoride, gallium carbonate, gallium acetate, gallium tartrate, gallium maltol, gallium oxalate, gallium formate, gallium oxide, hydrated gallium oxide, a coordination complex of gallium or a protein-bound gallium.

3. Use according to claim 2, wherein the coordination complex is a gallium pyrone, gallium pyridone, gallium hydroxymate, gallium aminocarboxylate or gallium oxime; and the protein is transferrin or lactoferrin.

4. Use according to any preceding claim, wherein the medicament is adapted for topical, transdermal, parenteral, intra-articular, gastrointestinal, trans-bronchial or trans-alveolar administration.

5. Use according to any preceding claim, wherein the tissue is selected from skin, cartilage, tendon, fascia and collagen-containing tissues that encapsulate organs.

## Patentansprüche

1. Verwendung einer Gallium-Verbindung zur Herstellung eines Medikaments, welches zur Bereitstellung von Gallium in löslicher Form in der Lage ist, zur Erhöhung der Binde- und Stützgewebewiederherstellung und -vermehrung, vorausgesetzt, daß das Gewebe nicht Knochen ist

2. Verwendung nach Anspruch 1, worin die Gallium-Verbindung Galliumnitrat, Galliumcitrat, Galliumphosphat, Galliumchlorid, Galliumfluorid, Galliumcarbonat, Galliumacetat, Galliumtartrat, Galliummaltol, Galliumoxalat, Galliumformiat, Galliumoxid, hydratisiertes Galliumoxid, ein Koordinationskomplex von Gallium oder ein Protein-gebundenes Gallium ist.

3. Verwendung nach Anspruch 2, worin der Koordinationskomplex ein Gallium-Pyron, Gallium-Pyridon, Gallium-Hydroxymat, Gallium-Aminocarboxylat oder Gallium-Oxim ist und das Protein Transferrin oder Lactoferrin ist.

4. Verwendung nach irgendeinem der vorhergehenden Ansprüche, worin das Medikament an eine topische, transdermale, parenterale, intraartikulare, gastrointestinale, transbronchiale oder transalveolare Verabreichung angepaßt ist.

5. Verwendung nach irgendeinem der vorhergehenden Ansprüche, worin das Gewebe aus Haut, Knorpel, Sehne, Fascie und Kollagen-enthaltenden Geweben, welche Organe einhüllen, ausgewählt ist.

## Revendications

1. Utilisation d'un composé de gallium pour la fabrication d'un médicament capable de délivrer du gallium sous forme soluble, pour renforcer la réparation et la croissance des tissus conjonctifs et de soutien, avec la condition que le tissu n'est pas de l'os.

2. Utilisation selon la revendication 1, dans laquelle le composé de gallium est le nitrate de gallium, le citrate de gallium, le phosphate de gallium, le chlorure de gallium, le fluorure de gallium, le carbonate de gallium, l'acétate de gallium, le tartrate de gallium, le maltol de gallium, l'oxalate de gallium, le formiate de gallium, l'oxyde de gallium, l'oxyde de gallium hydraté, un complexe de coordination de gallium ou du gallium lié à une protéine.

3. Utilisation selon la revendication 2, dans laquelle le complexe de coordination est une pyrone de gallium, une pyridone de gallium, un hydroxymate de gallium, un aminocarboxylate de gallium ou un oxyde de gallium ; et la protéine est la transferrine ou la lactoferrine.

4. Utilisation selon l'une quelconque des revendications précédentes, dans laquelle le médicament est adapté pour une administration topique, trans-dermique, parentérale, intra-articulaire, gastrointestinale, transbronchique ou trans-alvéolaire.

5. Utilisation selon l'une quelconque des revendications précédentes, dans laquelle le tissu est choisi parmi la peau, le cartilage, les tendons, l'aponévrose et les tissus contenant du collagène qui enveloppent les organes.
